# EUROPEAN PATENT APPLICATION

(11) **EP 1 178 288 A1**
(43) Date of publication of application: **06.02.2002**
(21) Application number: 01114238.7
(22) Date of filing: 12.06.2001
(51) Int. Cl.: G01D 7/02, G01D 7/08, G06F 3/14, G06G 7/60, A61B 5/02

(54) **User interface**

(30) Priority: 01.08.2000 SE 0002806
(71) Applicant: Siemens-Elema AB, 171 95 Solna 1 (SE)
(72) Inventor: Malmborg, Jessica, 10405 Stockholm (SE)

(57) **Abstract**

A user interface (2) for medical apparatuses, comprising a screen (12), a memory (14) holding normal data for at least two parameters, a signal input (20) for receiving signal data for the parameters and a control unit (18) devised to process normal data and signal data and generate a representation (30) of same on the screen (12), is described. The control unit (18) is devised to represent signal data for each parameter in the form of a sector in a regular polygon, compare signal data to normal data and vary the appearance of the sector according to the results of the comparison.

## Description

The present invention relates to a user interface according to the preamble to claim 1.

In step with technical developments in the software field and their application in many technologies, such as medical engineering, the amount of information facing system users is growing rapidly. User interfaces are becoming increasingly complex and harder to grasp. This is due in part to the fact that developments are governed by the opportunities offered by the new technologies and not by an understanding of the way people process information. Therefore, limitations in Man's ability to grasp and process information represent a risk when the amount of information increases. Information important to a medical treatment could be lost in other information and in a worst case scenario lead to errors.

US-3,811,040 describers a user interface for displaying physiological parameters. Each parameter is displayed as a vector on an axis and has a normal value selected so vectors have the same length when all the parameters are at the respective normal values. The vectors can be connected by a line to form a circle. If a value for any parameter deviates from the normal value, the circle is distorted along the axis displaying that parameter. Threshold values for each parameter can also be designated on respective axis.

The user interface according to the prior art has certain disadvantages. One is that legibility, despite the choice of the circular shape as a norm, is not good enough to enable an operator/monitor to quickly and effectively grasp information on a prevailing situation. So a deviation may not be reliably detected. A plurality of parameters can change in the same direction, so an unbroken circular shape might still be displayed. In addition, major deviations affect the circular shape to such an extent that determining whether parameters for adjacent axes have also changed might prove to be difficult.

Another disadvantage is that the visualisation does not offer any intuitive link to an interpretation of what is wrong and what countermeasures may be necessary. In principle, an unlimited number of circular shapes can be displayed on the screen, since the number of combinations of measurement values for the different axes is, in principle, unlimited. Nor is it easy for the operator to establish suitable priorities for different countermeasures on the basis of the figure displayed.

One objective of the present invention is to achieve a user interface that wholly or partially solves one or more of the aforementioned problems in the prior art.

This objective is achieved according to the invention when a user interface is devised as is evident from the characterising part of claim 1.

One main feature of the invention entails the generation by a control unit of a representation of signal data on a screen in the form of a regular polygon in which sectors, rather than axes, represent the parameters. However, the sectors do not necessarily have to coincide with the sides of the polygon. When signal data and normal data agree the polygon is regular. When signal data depart from normal data, the entire sector changes in one or more ways. When the change is the shape of the sector, it preferably changes uniformly. The polygon then loses its regularity in a specific way that is easy to grasp, even from a distance. The sector's colour, pattern or emphasis (e.g. it could start blinking at different rates), could also change, in addition to or instead of its shape.

Here, it would be highly advantageous for the operator if changes in a sector (or additional changes in an already changed sector) only occurred when a parameter value (signal data) exceeded/dropped below pre-set threshold values for the parameter. This would clarify changes exceeding pre-set threshold values for the parameter, whereas changes within those limits (regarded as less relevant to speedy assessment of the situation) are ignored. The number of changes in the representation can then be reduced (compared to continuous changes in relation to signal data). It is then easier for staff to see when no relevant changes have taken place and when changes have occurred. In particular the former makes it possible to avoid unnecessary checks on the representation. The latter makes it easier to intervene at an early stage and correct a situation before it progresses to the point at which an alarm is generated. Fewer alarms in a ward would create far fewer problems for both staff and patients (who inter alia can be stressed and awakened by alarms).

One particularly advantageous way of representing changes in a sector is to introduce uniform changes in the area along the midpoint rays delineating the sector. This would cause very distinct disruption in the regularity of the polygon's shape. This disruption of shape regularity is much more noticeable than a (slow) change in shape symmetry with retention of connected lines within the symmetrical shape.

Additional clarity is achieved when the sectors are displayed in colour. One colour for the basic image, when signal data and normal data coincide (e.g. green) and different colours for each stepwise change until the upper or lower alarm limit is reached (and e.g. red can be displayed). Simultaneous blinking could be one way to additionally designate some factor in this context, e.g. to show that a change took place very rapidly or that a composite interpretation of a plurality of minor deviations is deemed to be inherently serious, even if no individual parameter has reached an alarm limit yet.

The upper and lower alarm limits referred to here do not necessarily need to coincide with the alarm limits that generate acoustic alarms. One advantage of having different alarm limits is that staff can rectify a situation before an acoustic alarm is sounded. This would accordingly reduce the annoyance to staff and patients caused by frequent acoustic alarms. When the alarm limits do not coincide, the sectors can be assigned some colour other than red when a display alarm limit is reached. The colour can shift to red when an acoustic alarm limit is subsequently reached.

Upper and lower alarm limits are advantageously displayed on the screen as unbroken polygons inside and outside the basic image. The number of steps between the basic image and the respective alarm limit is preferably two. Fewer steps may not yield sufficient information in some cases. More than two steps may make the image more difficult to interpret from a distance, since the change could be difficult to decipher.

In this context it should be stressed that applicant views 'polygon' in a more broad respect than normally. The term shall therefore also refer to circles in the present application. The reason for this is that mathematically circles can be regarded as borderline cases for a sequence of inscribed polygons when the number of sides increases towards infinity.

The circle is a very useful cognitive shape in Man-machine interaction and therefore the preferred shape. Disruption of the circular shape caused by a change in the shape of a sector is probably one of the easiest changes for a person to detect. So any deviation can be detected at a great distance with only a quick glance.

For immediate access to additional information about a parameter, it would be advantageous if the screen were touch-sensitive. A simple touch in the sector representing the parameter would then yield immediate access to all necessary information about the parameter. Further, for parameters that can be set, a simple touch could also allow the operator to change the setting for the relevant parameter with no need to negotiate different menus and programming steps. If the size of the sector allows, all this information can be displayed within the sector. If the sector is not large enough, the information can take up a larger part of the screen. The basic image/deviation image (circle/polygon with or without changes in the appearance) can then be advantageously displayed on a reduced scale in a corner of the screen. A simple touch would then re-display this image full size. This would greatly simplify operation of the user interface.

Preferably, between 2 and 8 parameters should be displayed in a polygon or on a circle (even if more can be displayed). Ideally, 4 to 6 parameters are displayed. Additional polygons/circles can be used for increased access to additional parameters.

When several polygons/circles are used, it would be an advantage if the most important parameters were selected for a primary polygon/circle (being the one that is usually displayed). Alternately, one polygon/circle can display device-related parameters and another polygon/circle can display patient-related parameters.

Secondary polygons/circles could be arrayed as windows under the primary polygon. An operator can select them for on screen display to access them. They could also automatically be displayed on screen when deviations occur. When a plurality of polygons/circles display deviations, they can be arrayed according to degree of priority, with highest priority image being displayed on screen.

As an alternative to the windows model, secondary polygons/circles can be displayed on a reduced scale in suitable parts of the screen (e.g. in corners or along one side of the screen). An indication of which polygon presently being displayed in full size might then be appropriate.

Detailed embodiments of the user interface according to the invention will now be described, referring to the figures in which
FIG.1 shows a user interface, according to the invention, connected to a medical ventilator,
FIG. 2 shows an embodiment of the user interface,
FIG. 3 shows an example of a first representation of parameters that can be displayed on a screen in the user interface,
FIG. 4 shows an example showing how a first representation can display parameter deviations,
FIG. 5 shows an example of a second representation of parameters that can be displayed on a screen in the user interface, and
FIG. 6 shows an example of a third representation of parameters and the way in which a plurality of parameters can be displayed on the screen.

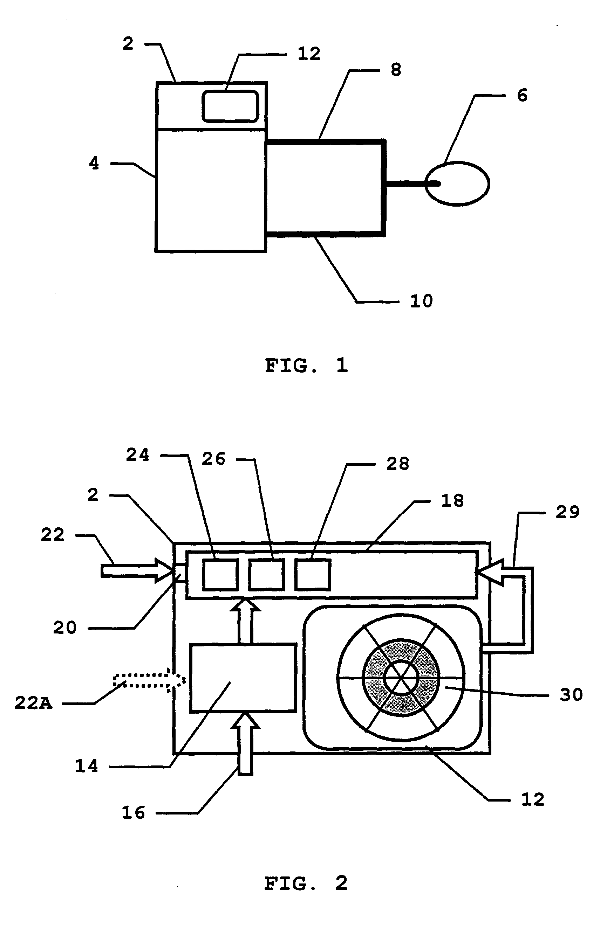

FIG. 1 shows a user interface 2 according to the invention operatively connected to a ventilator 4. The user interface 2 can be a fully integrated part of the ventilator 4 or detachably connected thereto.

The ventilator 4 can supply a patient 6 with breathing gas through an inspiratory line 8 and evacuate gas expired by the patient 6 through an expiratory line 10. Medical ventilators are well known in the medical art and need not be further described here. The ventilator 4 is only used as an example of a medical machine or apparatus for which the user interface according to the invention can be used. This makes it easier to describe and understand the composition and operation of the user interface.

A screen 12 is an important part of the user interface 2. Customary information (choice of breathing mode, parameters for the breathing mode and for the patient 6 etc.) can be displayed on it. Information can be input in one or more different (known) ways, e.g. with a keypad, knob, pushbutton, touch screen, microphone (voice-controlled), remote control (by cable, light, sound, radio etc.). Some information can be stored in memories (RAM or ROM). Some information can be retrieved from sensors in apparatuses (primarily the ventilator 4 but even other apparatuses such as a pulse oximeter, ECG-apparatus, etc.) and from sensors on the patient 6. All according to methods of treatment, diagnosis and collection of data well known in this context.

The screen 12 is also a key component in Man-machine interaction. It is here that the user interface 2 according to the invention improves and simplifies operation and monitoring of different systems in a new and revolutionary fashion.

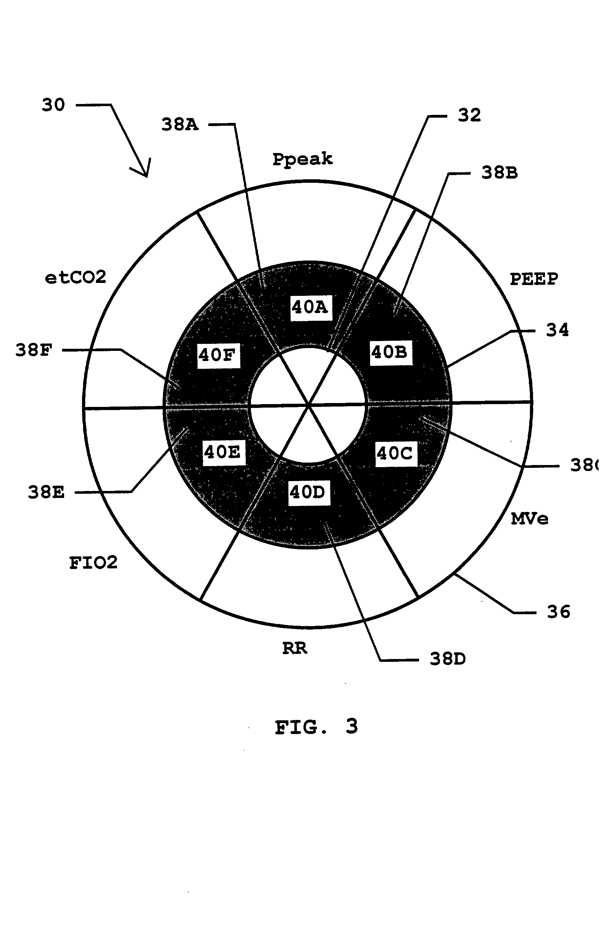

A more detailed drawing of one embodiment of the user interface 2 is shown in FIG. 2. The user interface 2 contains a memory 14. The memory 14 can have a component (ROM) holding permanently programmed information and a component (RAM) holding changeable information. The memory 14 contains inter alia normal data, representing expected values on certain parameters.

New information can be sent to the memory 14 across a first signal bus 16. The memory 14 is connected to a control unit 18. The control unit 18 receives signal data (primarily measurement data but even other data well, as is evident from the description below) via a signal input 20 from a second signal bus 22. Alternately, or as a complement, signal data can be read into the memory 14, illustrated in the figure by the dashed signal bus 22A.

In instances where normal data consist of a pre-set parameter, the value for the pre-set parameter can be sent straight to the control unit 18 (the setting itself constituting a 'memory'.)

The control unit 18 comprises the hardware and software necessary for subtracting signal data from normal data (illustrated with the subtractor 24) for each parameter, comparing the difference between one or more threshold values (illustrated with the comparator 26) and generating an image representing signal data and normal data according to the invention (illustrated with the image generator 28), shown as a circle 30, divided into sectors, on the screen 12.

In order to supply the most legible information possible, the image consists of a circle or regular polygon as long as signal data do not deviate excessively from normal data. In order to achieve this, normal data must be displayed for each parameter or represented by a sector at the same distance from the midpoint as other parameters. One advantage of the invention is that normal data can consist of both a fixed value (e.g. a pre-set value for a device function) or a range (e.g. a physiological measurement value such as carbon dioxide output). The advantage of the range is that more patients can be covered by 'normal' values for physiological measurements.

In the latter instance, the range limits can coincide with threshold values (for normal levels). Comparing signal data with threshold values may then be sufficient for determining the way in which the sector is displayed on screen. Naturally, signal data can also be subtracted from the range limits for normal data, and one or both of these can be compared with threshold values (only comparing values outside the range limit with threshold values is then sufficient).

It would also be advantageous for certain patient groups if 'new' normal data could be set or created. Certain parameters vary more than others, depending on the patient's condition. Individual patient data can therefore advantageously be used as normal data at various times. Normal data for a specific parameter will then vary over time with the patient's condition. New values set for normal data can naturally consist of a fixed value (e.g. the mean value over a specific period of time) or a range (e.g. based on variance or the extent of variance over a specific period of time).

Additional information on each parameter (or a plurality of parameters) can be easily obtained because the screen 12 is touch-sensitive (touch control). A light touch to one sector then transmits a control signal, across a third signal bus 29, from the screen 12 to the control unit 18, and an informative image is displayed for the parameter. Such display could for instance include present value of the parameter, a trend history covering a certain time, maximum and minimum values over a specific time, etc. This image is not shown in the figure since there are a multitude of well-known and obvious ways of displaying this particular information. A display consisting of pure textual information is perhaps the simplest and most obvious. The same information can also be shown in diagrams, histograms or similar.

With modern technology, realisation of the functions can be achieved in a plurality of ways. The physical components used do not need to be arranged in a common enclosure but can be disseminated. Functionality can also be shared. For example, the memory function and/or processor function can be shared with the apparatus (the ventilator 4 in this instance). One extreme would be for the user interface 2 to be completely integrated with the apparatus (ventilator 4). The other extreme would be for the user interface 2 to be completely independent and adaptable, enabling one single user interface 2 to work with a plurality of other apparatuses (ventilators, anaesthetic machines, X-ray equipment, ECG recorders, infusion devices, dialysis equipment etc.). The software can be stored on a hard disk, diskette, CD, DVD or some other medium.

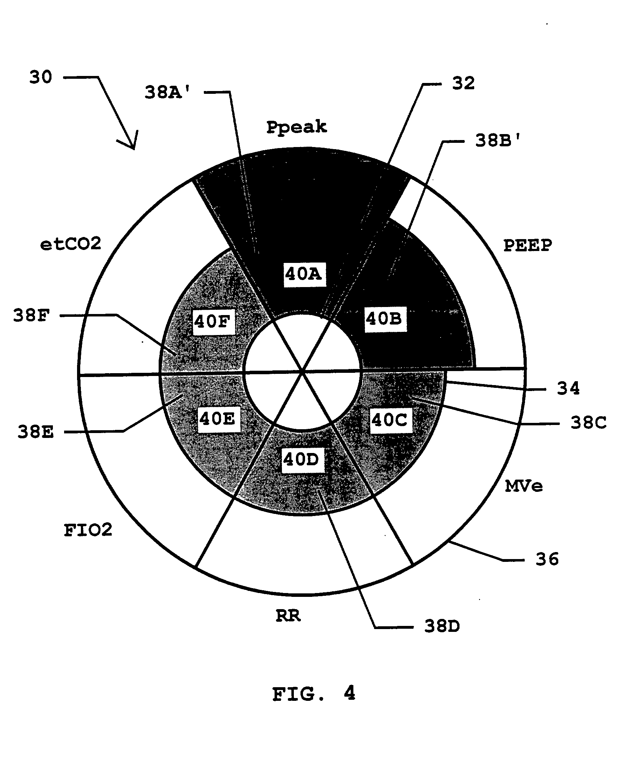

A first example of the way in which the representation generated by the control unit 18 on the screen 12 can look in a normal situation is shown in FIG. 3, and a representation with a deviation for two parameters is shown in FIG. 4. The following description therefore applies to both figures.

In this exemplified embodiment, the representation 30 consists of three circles, i.e. a lower alarm limit 32, a data circle 34 and an upper alarm limit 36. The data circle 34 in the example is divided into six sectors, one for each parameter to be displayed. As long as parameter values (signal data) are in accordance with normal data, a perfect circle is displayed. Since the user interface is described when used with a ventilator, the parameters have been selected on the basis of what may be appropriate for this application (more/fewer or some other selection of parameters is fully feasible, of course).

In this instance, a first sector 38A shows peak pressure during inspiration (Ppeak), a second sector 38B shows positive end-expiratory pressure (PEEP), a third sector 38C shows expired minute volume (MVe), a fourth sector 38D shows respiratory rate (RR), a fifth sector 38E shows oxygen content (FIO₂) and a sixth sector 38F shows end-tidal carbon dioxide content (etCO₂). Each parameter is represented by the area within the respective sector in the circle formed by the lower alarm limit 32 and the data circle 34. This area is shaded to indicate that it can be displayed in a different colour. For instance, all the sectors can be green as long as everything is normal. Green is usually perceived as indicating that everything is as it should be.

Actual measurement values for the parameters can be displayed in the sectors, as shown by the data fields 40A-F. Alternately, normal data can be shown in the data fields 40A-F, or (for apparatus constants) current settings. In the latter instance, normal data consist of a basic setting, and changes therein constitute signal data, i.e. are illustrated in the same way as when measurement data display excessive deviation. This can be useful in avoiding inadvertent changes in settings or for tailoring setting options to the patient's condition.

Deviation between signal data and normal data (preferably exceeding some threshold value) is shown as is evident from the versions of the first sector 38A' and the second sector 38B' in FIG. 4. The radius of the second sector 38B' has increased one step (and the colour changed). The radius of the first sector 38A' has increased three steps and reached the upper alarm limit 36. The stepwise change causes a distinct deviation from the circular shape right from the first step. Deviation is then readily noticeable by passing staff (and is made even more apparent by a change in colour which could be blue for the second sector 38B' and red for the first sector 38A'; a two-step change could be designated with e.g. yellow).

The change in the example from signal data coinciding with normal data to form circle 34 and the respective alarm limit 32, 36 takes place in two steps. Another number of steps (greater or fewer) is conceivable. The shift would be more gradual with additional steps. The number of steps for the respective parameter in the image can also vary. For example, the fifth sector 38E, which designates oxygen content, could jump straight to one of the alarm limits 32, 36 if oxygen content deviated from a normal range (e.g. ±5% of the pre-set value). The first sector 38A could have one intermediate step and other sectors two intermediate steps. Adaptation options are virtually endless. However, simplicity and information accessibility for the user is retained.

The corresponding applies when signal data are less than normal data and approach the lower alarm limit 32.

It is not necessary for the lower alarm limit 32 and the upper alarm lit 36 to coincide with the alarm limits that generate acoustic alarms. An essential advantage of the present invention is that a trend, in which a parameter approaches an acoustic alarm limit, is clarified in the image in a very distinct fashion, enabling staff to take steps before the alarm limit for an acoustic alarm is reached. Frequent (and often unnecessary) acoustic alarms are a very annoying feature of contemporary hospital environments. Staff, patients and visiting relatives are subjected to needless stress because of unnecessary acoustic alarms.

Displayed information can be managed in a number of ways. One way is to continuously represent changes in signal data in real time. A parameter displaying rapid, brief-duration change sequences would only cause brief changes in the sector (shape/colour). This is no problem as long as the brief changes are not of major importance to the operator/ monitoring staff. But when the display of even such brief changes is necessary, this can be accomplished in different ways. The deviation can be saved on a screen for a certain period of time (seconds/minutes/hours/number of breaths etc.) or until the deviation has been investigated by the operator/monitoring staff. The duration is prolonged if the deviation recurs. Recurrences can be indicated by an even larger deviation. Alternately, the colour, but not the shape, can change (or the reverse), or the sector could start blinking to indicate recurrences.

The above can also apply when signal data change more slowly or infrequently (e.g. once every respiratory cycle, such as certain ventilator/patient parameters).

Another possibility is to display trends, i.e. if a parameter increasingly approaches or exceeds a limit. An initial change in colour, followed by a change in shape (or the reverse) is also conceivable here. Alternately, a symbol in the form of an arrow can be inserted into the sector (indicating the direction of the trend).

In some instances, only a representation of the situation is of interest. Merely displaying the outer alarm limit and allowing it to constitute an alarm limit for all parameter deviations, i.e. both upward and downward, may then be sufficient. Outward changes to a sector (forming a larger sector) are more distinctive from a distance than a reduction in sector size. This also applies to parameters only capable of deviation in one direction.

One way to clarify inward changes towards the lower alarm limit (instead of reducing the size of the coloured area) is to change the sector more extensively. Assume that the reduction is displayed in two steps towards the lower alarm limit. As soon as the first step occurs, the sector is changed so it is delineated by a coloured area from the radius corresponding to the first step and out to e.g. the upper alarm limit.

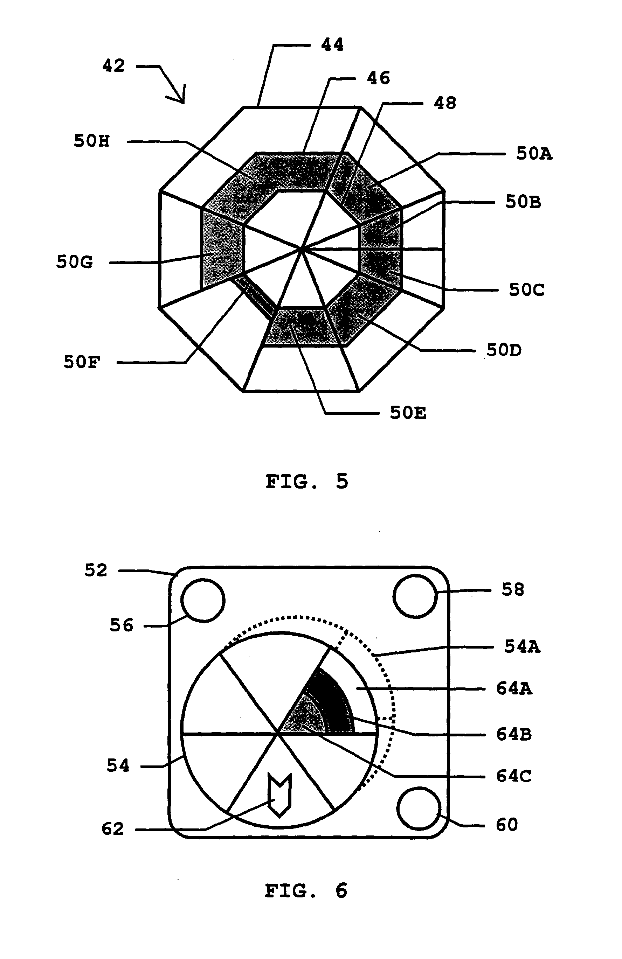

An alternative way of displaying the representation is shown in FIG. 5. In this instance, the representation 42 consists of three polygons. As in the preceding example, they correspond to an upper alarm limit 44, signal data 46 and a lower alarm limit 48.

The representation 42 is subdivided into eight sectors 50A-H. In this instance, the sectors 50A-H are not uniform. The second and third sectors 50B, 50C share one side of the polygon, whereas the eighth sector 50H comprises two sides. The choice of size for the sectors can be made on the basis of the importance of the sectors to be represented.

It should be noted here that the lines separating the sectors can be more or less pronounced. They can be omitted in extreme instances, especially when the sectors are uniform.

In the embodiment in FIG. 5, signal data for the parameter in the sixth sector 50F are smaller than normal data, and sector 50F has been reduced towards the lower alarm limit 48.

Additional examples of the way in which the control unit can generate representations are shown in FIG. 6. Four representations of signal data, designated 54, 56, 58 and 60, are displayed on screen. The representations 54-60 are positioned so all are visible, but only one of them (the first representation 54 in this instance) is enlarged. The positioning of the enlarged first representation 54 towards a corner of the screen 52 also clearly indicates the importance of the representation 54-60 which is currently enlarged (compared to the size the first representation 54 would have had if located in the centre of the screen 52).

The example also shows that no alarm limits are displayed on screen 52. Changes are only displayed with colours. One possibility is then for the entire sector to change colour, about the same way as described above. An indication of increasing or decreasing signal data can then be represented, if desired, with an arrow 62, as shown in the figure.

Alternately, the sector can be subdivided into zones, e.g. three zones 64A-C. The colour of zone 64A could change when values increase, and the colour of zone 64C could change when values decrease. Zone 64B could either have a constant colour or change colour to indicate some other information.

This information can be trend-related, i.e. zone 64B also changes colour if signal data have deviated from normal data over a long period of time (or frequently).

Other displayed information could show that the patient's condition as a whole (as reflected by signal data) requires the presence of a doctor or other staff, even if no individual parameter deviates enough to reach an alarm level. The middle zone for all sectors (or sectors relevant to the combined impact on the patient) can then change colour.

On the other hand, an individual parameter might occasionally reach an alarm limit without any risk to the patient (because a course of events occurred so rapidly or because other parameters failed to deviate from normal values). Sounding an alarm could then be unnecessary. The event can then be indicated with a blinking zone 64A or with another colour. The operator can then investigate the event at some other time.

If signal data in any of the other representations 56, 58, 60 change in such a way that staff should be alerted, the representation can automatically be enlarged (and partially overlap the first representation 54, or replace it entirely, whereupon the first representation 54 is displayed reduced in size).

With a simple touch, the operator can easily select the representation 54, 56, 58, 60 to be displayed at any moment. Another touch in the sector would yield immediate access to information on the parameter.

An alternative version of several representations is also indicated in FIG. 6. Instead of several small and one large representation, the representations can be in the form of windows superimposed upon one another. This is indicated with a dashed representation 54A under the first representation 54.

As in the above, the control unit can contain a function that automatically places the representation 54, 54A with the greatest deviation, most deviations or highest priority deviations at the top of the stack.

Manual changes can be made e.g. by touching the middle of the representation 54 or alongside the representation 54 in order to browse through all representations 54, 54A.

A few examples are described above. Other examples are conceivable, of course. For example, the number of sides in a polygon can vary (from 3 and up), even if only an octagon is displayed. Multiple screens can be used when a plurality of representations needs to be displayed. In many instances, multiple screens are already being used by each hospital bed. Separate screens for the representations are also possible (other data and information being displayed on other screens.)

Combinations of the presented examples are also possible. For example, the sectors in displayed circles can vary in size (different angles between the radii forming each sector), and the sectors in the octagon can be of equal size (e.g. corresponding to the length of the sides). The colour and changes in shape described for one version are applicable to other versions.

The choice of the number of parameters and which parameters to display naturally depends on each situation. The example shown in FIGS. 3 and 4 relate to parameters suitable for volume-controlled breathing from a ventilator. Other parameters (more or fewer) are also conceivable for this mode. A completely different choice may be appropriate for other breathing modes. Anaesthesia requires the monitoring of other parameters in this way. Other medical apparatuses demand other parameters. The main advantage of the user interface according to the invention is that it is particularly adaptable to the human brain's ability to perceive and process information. The user interface provides intuitive, simple and immediate comprehension of the prevailing situation.

## Claims

1. A user interface (2) for medical apparatuses (4), comprising a screen (12; 52), a memory (14) holding normal data for at least two parameters, a signal input (20) for signal data on the parameters and a control unit (18) devised to process normal data and signal data and generate a representation (30; 42; 54) of same on the screen (12; 52), **characterised in that** the control unit is devised to represent signal data for each parameter in the form of a sector (38A-F; 50A-H) in a regular polygon (34; 46; 54), compare signal data to normal data and vary the appearance of the sector (38A-F; 50A-H) according to the results of the comparison.

2. The user interface according to claim 1, **characterised in that** the control unit (18) is devised to vary the appearance of the sector (38A-F; 50A-H) only if the difference between normal data and signal data exceeds a pre-set threshold value for the parameter.

3. The user interface according to claim 1 or 2, **characterised in that** the control unit (18) is devised to vary the area of the sector (38A-F; 50A-H) in such a way that a clear distinction is developed between it and adjacent sectors (38A-F; 50A-H).

4. The user interface according to claim 3, **characterised in that** the control unit (18) is devised to vary the area of the sector (38A-F; 50A-H) in such a way that the area is increased if signal data are greater than normal data and decreased if signal data are less than normal data.

5. The user interface according to any of claims 2-4,
**characterised in that** the control unit (18) is devised to generate an inner regular polygon (32; 48) corresponding to a lower alarm limit for the parameters.

6. The user interface according to any of claims 2-5, **characterised in that** the control unit is devised to generate an outer regular polygon (36; 44) corresponding to an upper alarm limit for the parameters.

7. The user interface according to claim 5 or 6, **characterised in that** the control unit (18) is devised to vary the sectors (38A-F; 50A-H) stepwise towards the alarm limit (32, 36; 44, 48) in a specific number of steps.

8. The user interface according to claim 7, **characterised in that** the number of steps is two.

9. The user interface according to any of the above claims, **characterised in that** the sectors (38A-F; 50A-H) are generated in colour, and the colour is varied according to the results of the comparison between normal data and signal data.

10. The user interface according to any of the above claims, **characterised in that** the regular polygon consists of a circle (30; 54).

11. The user interface according to any of the above claims, **characterised in that** the screen (12) has a touch-sensitive surface, and the control unit (18) is devised to generate, when a sector (38A-F; 50A-H) is touched, an image containing more detailed information on the situation for the parameter to which the sector (38A-F; 50A-H) corresponds.

12. The user interface according to any of the above claims, **characterised in that** the control unit (18) is devised to generate additional regular polygons (54, 56, 58, 60; 54A).

13. The user interface according to claim 12, **characterised in that** the regular polygons (54, 54A) are stacked atop one another, and the polygon with the greatest deviation is automatically placed at the top of the stack.

14. The user interface according to claim 12, **characterised in that** the on the screen (52) are displayed all the regular polygons (54, 56, 58, 60) in a small format and one of these (54) in a larger format.
